# EUROPEAN PATENT SPECIFICATION

(11) **EP 3 593 356 B1**
(45) Date of publication and mention of the grant of the patent: **17.09.2025**
(21) Application number: 18711266.9
(22) Date of filing: 08.03.2018
(51) Int. Cl.: G16H 50/20, G16H 50/30, G16H 40/63, A61B 5/0205, A61B 5/00, A61B 5/024, A61B 5/145

(54) **PATIENT STATUS MONITOR WITH VISUALLY STRONG PATIENT STATUS DISPLAY**
PATIENTENZUSTANDSMONITOR MIT VISUELL STARKER PATIENTENZUSTANDSANZEIGE
ÉCRAN D'ÉTAT DE PATIENT COMPORTANT UN AFFICHAGE D'ÉTAT DE PATIENT VISUELLEMENT FORT

(30) Priority: 10.03.2017 US 201762469549 P
(43) Date of publication of application: 15.01.2020
(73) Proprietor: Koninklijke Philips N.V., 5656 AG Eindhoven (NL)
(72) Inventor: HENDRICKSON, Maria, Fay, 5656 AE Eindhoven (NL)
(74) Representative: Philips Intellectual Property & Standards
(86) International application number: PCT/EP2018/055769
(87) International publication number: WO 2018/162644

(56) References cited:
- WO-A2-03/102850
- US-A1- 2003 146 942
- US-A1- 2013 253 348
- US-A1- 2015 025 405
- US-A1- 2015 356 262

## Description

### FIELD

The following relates generally to the patient monitoring arts, patient status monitoring arts, respiratory monitoring arts, cardiovascular monitoring arts, neurological monitoring arts, computer icon generation arts, and related arts.

### BACKGROUND

One of the key responsibilities of clinicians (e.g. nurses, doctors, or so forth treating patients in a hospital or other clinical setting) is to monitor and evaluate a status of the patient. However, this monitoring and evaluation of the patient is challenging, particularly in the case of patients in an intensive care units (ICU), Cardiac Care unit (CCU), or other critical care unit. The clinician in the critical care unit is bombarded with large volumes of patient data from multiple sources. This data comes from devices, from other systems, and from patient assessments. In one study, it was found that on an average 1341 items of clinical data was charted in an adults ICU chart per day (*see* Manor-Shulman O, Beyene J, Frndova H, Parshuram CS. Quantifying the volume of documented clinical information in critical illness. J Crit. Care 2008; 23(2): 245-250.). The same study found this increased up to 2354 for children receiving certain therapies. This challenge is further expressed in the DIKW pyramid which describes the transformation of data into information into knowledge and then into wisdom (*see* Henry, Nicholas L. (May-June 1974). "Knowledge Management: A New Concern for Public Administration". Public Administration Review 34 (3): 189. doi:10.2307/974902. JSTOR 974902). Clinical evaluations and decisions cannot be made effectively or efficiently until data has been transformed into information or knowledge. Often this is done by the clinician themselves, which can lead to delays and/or errors. Some healthcare information systems present the deluge of data in an automated format, but as raw data without substantial post-processing.

Some systems do transform data into information and knowledge, thereby reducing the cognitive load on the clinician and making healthcare decisions more effective. In one approach, the patient status monitoring system displays a "dashboard" for each patient, which includes a row or matrix of body system icons for the patient in a bed. Each body system icon indicates the status of a corresponding body system (e.g. cardiovascular system, respiratory system) with a color. In one conventional color scheme, white indicates normal, yellow indicates abnormal, and red indicates a severe impairment. The indication of patient status using body system icons is an approach often found in clinical information systems. The health status of a patient is suitably determined by applying clinical decision support (CDS) algorithms developed by appropriate medical experts or medical domain-specific medical organizations. The patient status monitor may monitor a large number of patients, e.g. every patient in the critical care unit, using a separate dashboard of body system icons for each patient in the critical care unit.

WO 03/102850 A2 discloses a system and method for gathering and displaying information in data intensive environment. It is particularly concerned with data analysis in a critical care environment to provide a graphical display of derived information, comprising a series of bar charts representative of a corresponding series of functions.

US 2013/253348 A1 discloses a clinical decision support system for patient treatment having a monitoring device operably coupled to a patient, wherein the monitoring device outputs a monitoring signal in response to a measured parameter of the patient.

US 2015/356262 A1 discloses a system and method for providing a user engagement platform to support clinical decisions, wherein the user engagement platform is configured to receive and process data parameters obtained from a patient electronic health record and identify abnormalities based on such data parameters.

US 2003/146942 A1 discloses a graphical user interface presenting a clinician/user coded medical information in at least one logical hierarchy, and accepting selections of the presented information made by the user.

The following discloses new and improved systems and methods to overcome these problems.

### SUMMARY

The invention is defined by the independent claims. The dependent claims define advantageous embodiments.

One advantage resides in providing a patient status monitor with a patient status icon having a strong visual that can used in displays containing small number of patients, or optionally on displays that monitor patient populations.

Another advantage resides in providing a patient status icon that is obscure enough to the general public so that it could be used on white boards and can address concerns patient confidentiality.

Another advantage resides providing in a patient status icon with sections that visually map to the locations of body systems, thereby facilitating rapid comprehension of the constituent body systems or physiologies being represented.

Another advantage resides in providing a patient status icon that provides a strong visual representation for those body systems particularly at risk.

Another advantage resides in providing a patient status icon with various shapes to distinguish different types of patients or patients at different treatment phases.

Another advantage resides in providing a patient status icon with additional visuals indicating therapies being administered to a patient.

Another advantage resides in providing a patient status icon with selectable features to find for more details on that body system corresponding to the selected section.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the invention.
FIGURE 1 diagrammatically shows a patient status icon according to one aspect.
FIGURES 2A and 2B diagrammatically show the patient status icon of FIGURE 1 with different sections shaded.
FIGURE 3 diagrammatically shows a device for measuring respiration of a patient according to one aspect.
FIGURE 4 diagrammatically shows an operational flow chart for generation of the icon of FIGURE 1.
FIGURES 5A-5D diagrammatically show medical therapy icons on the icon of FIGURE 1.
FIGURES 6A-6E diagrammatically show different shapes of the icon of FIGURE 1.
FIGURE 7 diagrammatically shows a display screen with patient information from which the icon of FIGURE 1 can be shown.
FIGURE 8 diagrammatically shows a selected patient of FIGURE 7 with the corresponding patient status icon of FIGURE 1.

### DETAILED DESCRIPTION

The following relates to a new patient status icon, which is designed to be more readily interpreted when displayed on a small scale, such as in the context of dozens of similar icons on a white board or nurses' monitoring station overview screen for a large medical facility. Existing icons employ known symbols representing various body systems (e.g., brain/neurological; cardiac; respiratory; etc.) which may be color coded to represent normal, abnormal, or critical (e.g. white, yellow, red respectively).

Current icon systems have some disadvantages. Some information systems manage the care of intensive care patient populations, often in the hundreds. However, the body system icons are not visually strong enough to identify patients at risk within a population display. The physician reviewing the screen must be able to review across multiple patients and identify those that need special attention. However, the row of body system icons of each patient bed dashboard must be reduced in size to fit a large number of patients on the display. In this reduced size, it can be difficult for the nurse or physician to isolate and recognize a single body system icon of a single patient which is indicating a clinically urgent problem.

Another concern relates to patient privacy. Most care units in the hospital have a white board posted on the wall near the central station listing the patients, their rooms, and important information such as age, gender, and health status. It is a valuable tool for team communication, for example enabling key information to be transferred during nurse shift changes or when a physician visits patients in the care unit. Updating the white board manually is laborious and can be error prone. Leveraging the patient status monitor to display an electronic "white board" in which the status of each patient is represented by the corresponding bed icon would be advantageous. Again, however, existing dashboards of patient status monitors do not provide visually strong representations. A "visually strong" representation should convey the patient information in a concise manner that is easily interpreted without detailed examination (by reading the representation at a glance) and conveys critical information such as indications of body systems or physiologies in critical condition, identification of critical therapies being administered (mechanical ventilation, vasopressor therapy, or so forth), and general patient information such as the status (pre-operative, post-operative, et cetera) in a manner that can be grasped in an instant as the nurse or doctor scans the display.

The disclosed patient icon uses a geometrical shape that maps to the layout of the human body in the coronal plane. The coronal plane is the standard anatomical plane that divides the body into dorsal and ventral (bank and front, or posterior and anterior) portions. The coronal plane is sometimes referred to as the frontal plane. In the coronal plane, the heart (the most critical component of the cardiovascular system) can be viewed as being centrally located, with the head (containing the brain, i.e. the most critical component of the neurological body system) located above the heart, the left and right lungs (the most critical components of the respiratory system) located to opposite sides of the heart. In accord with this mapping of critical organs in the coronal plane, the illustrative patient icon places the cardiac and respiratory regions centrally and larger compared with other regions to emphasize these life-critical body systems. (Making the cardiac and respiratory regions larger may not reflect actual relative organ sizes which can vary amongst individuals, but does reflect the criticality of these life-critical body systems). The resulting icon is more easily read when reduced to small scale, and the layout alignment with the human body improves the intuitiveness of the icon. In some embodiments, the state of one or more physiologies may also be represented, e.g. boundary lines to represent hematology status.

Another optional aspect of the disclosed patient status icon is to represent therapies, preferably using graphical representations of fluid flow or other therapeutic activity (e.g. a constriction shown to illustrate vasopressor therapy). Another optional aspect is the use of a white color to indicate a normal body system contrasted with a background color to represent a system whose status is uncertain. This is useful since knowledge that a system is normal versus unknown can materially affect medical decision-making. There may also be various icon shapes to represent various medical workflow states, e.g. pre-operative versus post-operative, or variants to represent specific medical categories, e.g. an extra central portion representing the status of the fetus in the case of pregnant patient.

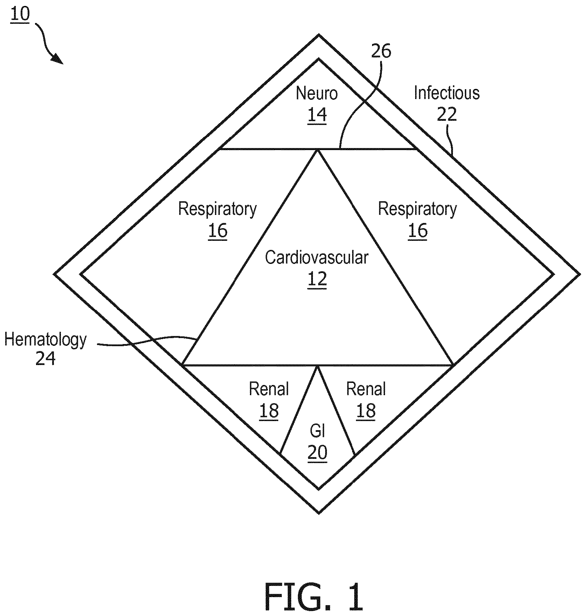

With reference to FIGURE 1, an illustrative patient status icon **10** for displaying a status of one or more body systems or physiologies of a patient is shown. As shown in FIGURE 1, the patient status icon **10** includes a plurality of sections or areas **12, 14, 16, 18, 20,** and **22.** Each of these sections correspond to a respective body system or pathology of the patient (e.g., cardiovascular body system, respiratory body system, neurological body system, gastrointestinal (GI) body system, renal body system, hematology physiology, systemic infection physiology, and the like). For example, the patient status icon **10** includes at least a cardiovascular icon section **12,** a neurological icon section **14,** and a respiratory icon section **16.** As shown in FIGURE 1, the cardiovascular icon section **12** corresponds to the cardiovascular body system and comprises an area that is centrally located on the icon **10.** The neurological icon section **14** corresponds to the neurological body system, and comprises an area that is located above the cardiovascular icon section **12.** The respiratory icon section **14** corresponds to the respiratory body system, and includes left and right sub-sections comprising areas which are located left and right, respectively, of the cardiovascular icon section **12.**

The illustrative patient status icon **10** also includes a gastrointestinal (GI) icon section **18** corresponding to the gastrointestinal body system. The GI icon section **18** comprises an area located below the cardiovascular icon section **12.** A renal icon section **20** corresponding to the renal body system is also located below the cardiovascular icon section **12,** i.e. comprises an area located below the cardiovascular section **12.** The illustrative GI icon section **18** includes left and right sub-sections comprising areas located left and right, respectively, of the renal icon section **20.**

Some sections may be embodied as other than area features of the icon. For example, the illustrative patient status icon **10** further includes a systemic infection icon section **22** corresponding to the systemic infection physiology - the systemic infection icon section **22** comprises an outer border of the patient status icon **10,** which graphically captures the physiology of a systemic infection which typically infects a substantial portion, or even all, of the patient's body tissues. As another example, a hematology icon section **24** corresponding to the hematology physiology comprises interior border lines **26** of the patient status icon **10.**

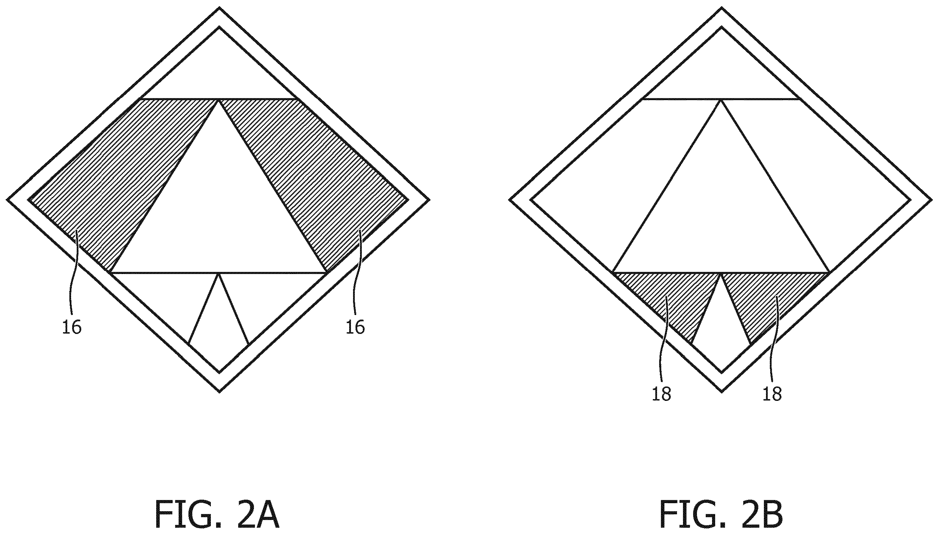

Advantageously, the cardiovascular icon section **12,** the neurological icon section **14,** and the respiratory icon section **16** are larger in area than the other icon sections **18, 20, 22,** and **24** to emphasize these life-critical body systems. As shown in FIGURES 2A and 2B, when any of the icon sections 12, **14, 16, 18, 20, 22,** and **24** are filled in with a color representative of a state of the respective body system or physiology (as described in more detail below), the filled-in area of the life-critical icon sections **12, 14,** and **16** is more prominent (FIGURE 2A) than the filled-in area of the other icon sections **18, 20, 22,** and **24** (FIGURE 2B). The overall collection of information related to these body systems and physiologies are deemed critical to short-term survival of the patient. For example, if any the icon sections are color-coded to show that a status of the corresponding body system or physiology is critical, then death or permanent impairment the corresponding body system or physiology of the patient is possible.

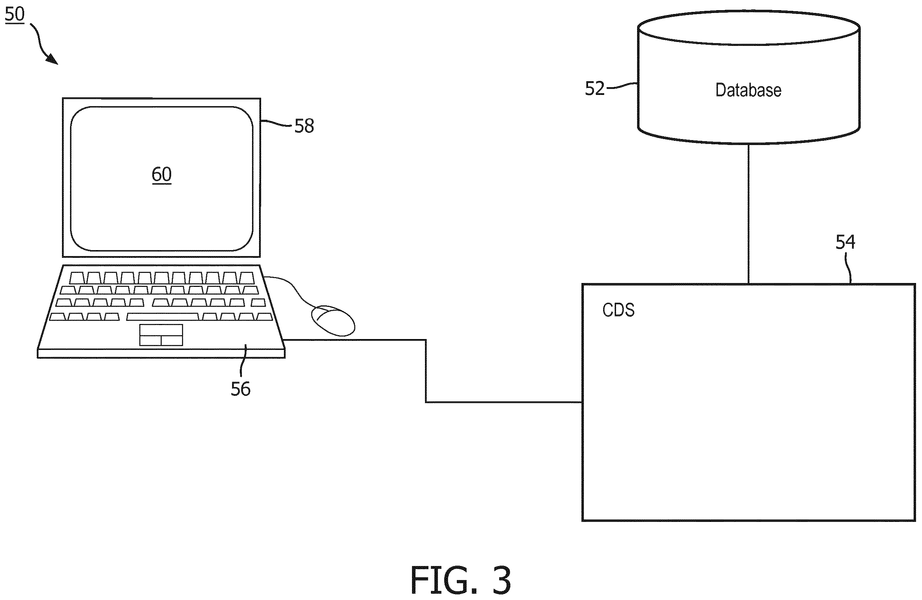

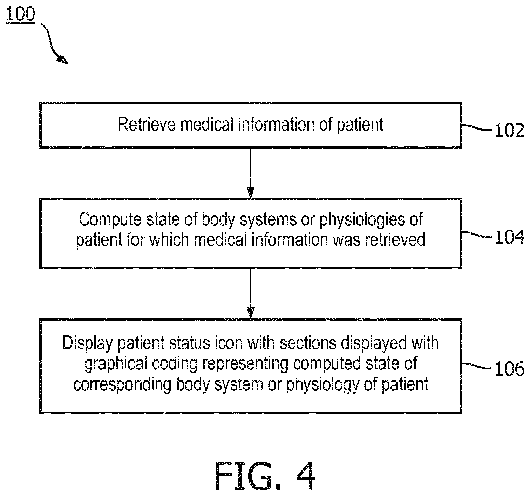

The patient status icon **10** is generated with a patient status monitoring method **100** (FIGURE 4) performed by a patient status monitoring apparatus **50** (FIGURE 3). Referring first to FIGURE 3, the patient status monitoring apparatus **50** includes or has (at least) read access to at least one electronic patient medical database **52,** such as an Electronic Medical Record (EMR) database, Electronic Health Record (EHR) database, cardiovascular information system (CVIS), various combinations thereof, or so forth. The database **52** stores and contains medical information of the patient, including at least neurological body system information of the patient, cardiovascular body system information of the patient, and respiratory body system information of the patient. The database **52** may also contain medical information related to systemic infection physiology of the patient, gastrointestinal body system information of the patient, renal body system information of the patient, and hematology physiology information of the patient.

The medical database **52** contains data relevant to assessing the state of various body systems (cardiovascular, respiratory, neurological, et cetera) and physiologies (hematology physiology, systemic infection physiology, et cetera). However, this data is not directly usable to assess the states of those various body systems and physiologies.

To generate body system and physiology state information from the medical data contained in the medical database **52,** the patient status monitoring apparatus **50** also includes a clinical decision system (CDS) **54** configured to apply clinical decision support (CDS) processing to compute a state of each of the body systems and physiologies of the patient, as described in more detail below. At least one processor **56** (implemented on, for example, a computer **58**) is programmed to generate the patient status icon **10** based on the computed states of the body systems and physiologies. A display device **60** (e.g., of the computer **58**) is configured to display the generated patient status icon **10** for use by a medical professional.

With reference to FIGURE 4, a patient status monitoring method **100** suitably performed by the patient status monitoring apparatus **50** is described. At **102,** medical information of the patient is retrieved from the at least one database **52.** At **104,** a state for each of a plurality of body systems or physiologies including at least a neurological body system, a cardiovascular body system, and a respiratory body system is computed by applying clinical decision support (CDS) processing with the CDS **54** to the retrieved medical information. At **106,** the patient status icon **10** with a plurality of icon sections **12, 14, 16, 18, 20, 22,** and **24** corresponding to the plurality of body systems or physiologies is displayed on the display device **60,** with each icon section being displayed with graphical coding that represents the computed state of the corresponding body system or physiology of the patient.

At **102,** medical information is retrieved from the database **52,** which can include not only information of the cardiovascular body system, respiratory body system, neurological body system, but also the gastrointestinal (GI) body system, renal body system, hematology physiology, systemic infection physiology, and the like, of the patient. The particular information retrieved from the database **52** depends on the inputs called for by the CDS processing performed by the CDS **54.**

At **104,** the CDS **54,** using CDS processing, computes a state of each body system or physiology for which information was retrieved from the database **52.** For example, the CDS processing can output, for each body system or physiology, a state belonging to a closed set of possible states, e.g. a closed set including at least a first state, a second state, a third state, and a fourth state. In the illustrative example, the first state indicates that a status of the patient represented by the corresponding section **12, 14, 16, 18, 20, 22,** and **24** of the icon **10** is normal. The second state indicates that a status of the patient represented by the corresponding section **12, 14, 16, 18, 20, 22,** and **24** of the icon **10** is abnormal but not critical (i.e., non-life threatening, or having no or low probability of leading to a permanent debilitation). The third state indicates that a status of the patient represented by the corresponding section **12, 14, 16, 18, 20, 22,** and **24** of the icon **10** is critical (i.e., life threatening, or having a significant possibility of leading to permanent debilitation). The fourth state indicates that a status of the patient represented by the corresponding section **12, 14, 16, 18, 20, 22,** and **24** of the icon **10** is unknown. This is merely an illustrative system for assigning states to the body systems and physiologies, and other state definitions are contemplated. In general, the number of possible states should be low, e.g. no more than two to four or five states at most, as having an excessive state space makes interpretation of the patient status icon more difficult.

The respective states are computed based on a set of predefined CDS rules developed using clinical information such as may be obtained from studies, and/or guidelines published by medical organizations in their areas of competency (e.g., cardiovascular guidelines published by the American Heart Association or so forth). In one non-limiting illustrative example, for the cardiovascular body system, the following CDS processing may be employed. If the retrieved information indicates that the patient's heart rate is greater than 160 beats per minute or below 40 beats per minute, or if the mean blood pressure of the patient is less than 50 mmHg, then the cardiovascular body system is in a critical (i.e., third) state. In another non-limiting illustrative example, for the respiratory body system, if the respiratory rate is greater than 30 breaths per minute, or less than 5 breaths per minute, or if the oxygen saturation of the patient is less than 85 %, then the respiratory body system is in a critical state. In another non-limiting illustrative example, if the respiratory rate is between 25-30 breaths per minute, or between 5-10 breaths per minute, then the respiratory system is in an abnormal (i.e., second) state. In another non-limiting illustrative example, if the patient's body temperature is greater than 38 C, the patient's white blood cell count is greater than 15,000, or the patient's white blood cell count is less than 3,000, then the infectious physiology is in the abnormal (i.e., second) state. In yet another non-limiting illustrative example, if the patient's hematocrit level is less than 19%, or if the patient's hemoglobin level is less than 7 g/dL, then the hematology system of the patient is in the critical (i.e., third) state. These are merely non-limiting illustrative examples, and more generally the CDS **54** preferably implements CDS guidelines embodying the most recent clinical knowledge and expertise for assessing the state of each body system or physiology. In some embodiments, the CDS **54** implements CDS guidelines specific for the class or category of patient being monitored, e.g. employing pediatric CDS guidelines for pediatric patients, guidelines specific for a patient with a certain chronic condition (e.g. a patient with chronic heart failure), or so forth.

The fourth state (indicating the status of the body system or physiology is unknown) is assigned if the CDS rule(s) cannot be executed to determine the state of that body system or physiology due to missing information needed to generate the state. For example, in assessing the state of the systemic infection physiology, the white blood cell count may be needed information; thus, if the white blood cell count is unavailable then the systemic infection physiology is suitably assigned the fourth state indicating that it is not known whether the patient has a systemic infection.

The CDS rules may include numerous features known in the art for providing more accurate and robust assessment of body systems and physiologies. For example, the CDS rules may include rules for selecting or combining measurements of the same physiological parameter. Thus, for example, if the heart rate is available from electrocardiogram (ECG) and pulse oximeter, the CDS rule may select the ECG reading as likely to be more accurate, or alternatively may average the heart rates output by the ECG and pulse oximeter, or may choose one or the other based on a quality metric such as signal-to-noise ratio (SNR) of the ECG and photoplethysmography signals. The CDS rules applied by the CDS **54** are also preferably updated as appropriate to reflect current medical knowledge and practice, e.g. to implement the latest cardiovascular assessment guidelines. (These updates may be manual CDS firmware or software updates or the like).

At **106,** once the state of each corresponding body system or physiology of the patient is computed, the icon **10** is generated by the processor **56.** The icon **10** is generated so that, when it is displayed on the display device **60,** the sections **12, 14, 16, 18, 20, 22,** and **24** are displayed with graphical coding comprising a color that represents the computed state of the corresponding body system or physiology of the patient. In an illustrative example, the graphical coding complies with a coding scheme in which white represents a normal computed state of the corresponding body system or physiology (i.e., the first state), yellow represents a non-serious abnormal computed state of the corresponding body system or physiology (i.e., the second state), red represents a serious abnormal computed state of the corresponding body system or physiology (i.e., the third state), and a background color other than white, yellow, or red represents an indeterminate computed state of the corresponding body system or physiology (i.e., the fourth state). When the icon **10** is shown on the display device **60,** each of the sections **12, 14, 16, 18, 20, 22,** and **24** for which information was retrieved for is displayed with a color representing one of the four states. Based on the respective colors, information about the patient can be determined by a medical professional. While color coding is a preferred graphical coding scheme, other types of graphical coding are contemplated to be employed in the alternative or in addition to color coding. For example, a section that is in the third (most urgent) state may be displayed as a flashing element (cycling on/off), or displayed with color cycling, or displayed using a brighter intensity, so as to emphasize the urgent state of that body system or physiology. By contrast, if the section is in the first (normal) state then it may optionally be displayed at lower intensity to provide further de-emphasis. These are merely illustrative optional features.

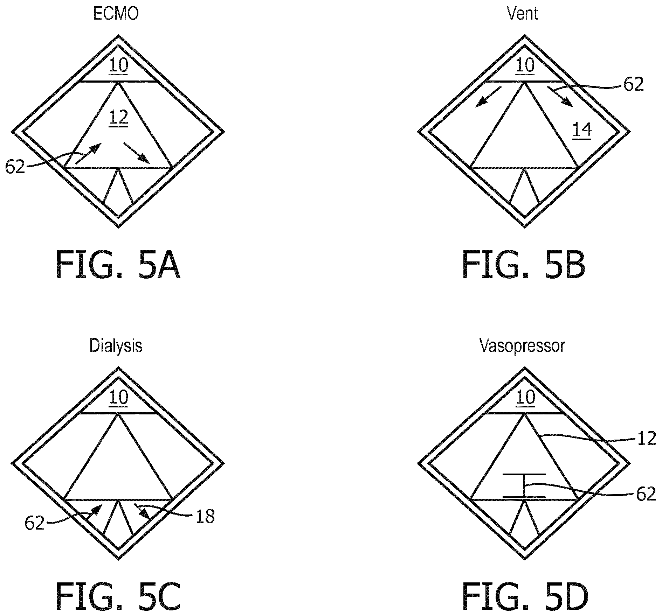

In some examples, during generation of the patient status icon **10,** therapy information about the patient can be displayed on the icon. (As used herein, the term "medical information" encompasses information about medical therapies delivered to the patient). For example, from the retrieved patient medical information from the database **52,** a medical therapy being delivered to the patient to treat a body system or physiology of the patient undergoing treatment is identified. When a medical therapy is identified, the icon **10** is displayed on the display device **60** and annotating an icon section **12, 14, 16, 18, 20, 22,** and **24** corresponding to the body system or physiology undergoing treatment with a medical therapy indicator **62** representing the medical therapy. For example, in FIGURES 5A-5D, several examples of medical therapy indicators **62** are illustrated as arrows representing a fluid flow through the patient produced by the medical therapy. In FIGURE 5A, arrows **62** are shown in the cardiovascular icon section **12,** and are indicative of extracorporeal membrane oxygenation (ECMO) therapy where blood is removed from the body, oxygenated, and returned to the body. In FIGURE 5B, arrows **62** are shown in the respiratory icon section **16** and are indicative of mechanical ventilator providing oxygenated gas to the patient's lungs. In FIGURE 5C, arrows **62** are shown in the renal icon section **18** and are indicative of renal dialysis. In FIGURE 5D, arrows **62** are shown in the cardiovascular icon section **12** and are indicative of vasopressor therapy. The overall collection of information related to these therapies are deemed critical to short-term survival of the patient, and does not include every therapy being applied to the patient. For example, if any the icon sections are include an indicator **62** to show that the corresponding body system or physiology of the patient is critical, then death or permanent impairment of that corresponding body system or physiology of the patient is possible.

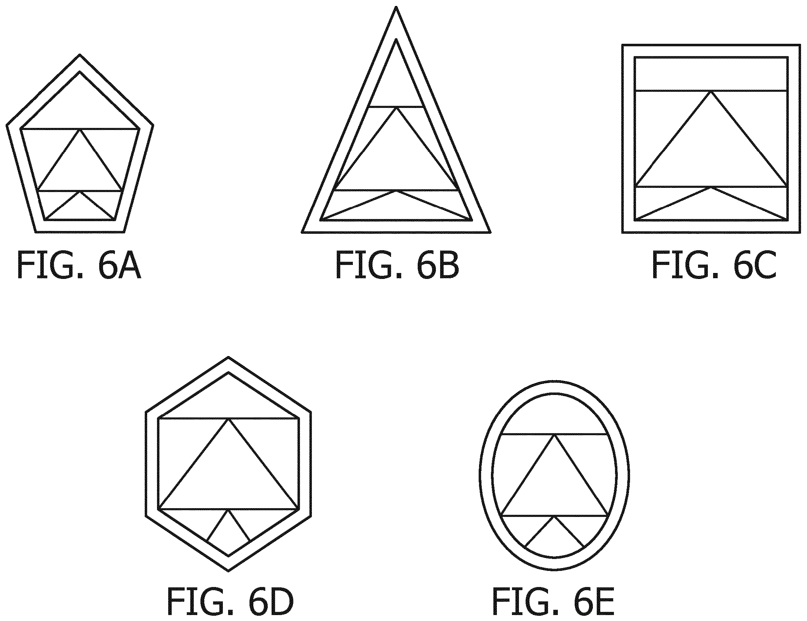

In other examples, a shape of the patient status icon **10** can be changed based on a stage of treatment. As shown in FIGURE 1, the icon **10** is shown as a diamond shape. As shown in FIGURES 6A-6E, the patient status icon **10** can have a pentagonal shape, a triangular shape, a square shape, a hexagonal shape, and an elliptical shape. Each of these shapes can be indicative of a stage of treatment of the patient. One of these six shapes can correspond to: (1) a pediatric patient; (2) an adult patient; (3) a pre-operative patient; (4) an operative patient; (5) a post-operative shape; or (6) a women undergoing labor and delivery where the pre-delivery phase is one shape and the post delivery is another gem shape.

As a further optional feature, each section of the patient status icon **10** is user-selectable, e.g. by clicking or double-clicking on the section using a mouse, trackpad, or other pointing-type user input device. As another selection alternative, the display **60** may be a touch-sensitive display, in which case the user can touch the icon section shown in the display **60** to select it. Upon selecting the icon section, context-sensitive information pertaining to the corresponding body system or physiology is brought up in a pop-up window or other display region on the display **60.** In similar fashion, selection of one of the medical therapy indicators **62** may bring up a display of context-sensitive information on the therapy (e.g., selecting the mechanical ventilator indicator **62** shown in the example of FIGURE 5B may bring up a pop-up window showing current ventilator mode and settings).

The patient status monitor may take various form factors with the data processing operations variously distributed. For example, the patient status monitor may be a nurses' station and/or an electronic white board, with the CDS and icon generation processing being performed locally on the nurses' station monitor processor and/or remotely at a hospital server in communication with the nurses' station via a wired or wireless hospital data network (e.g. wired and/or wireless Ethernet, WiFi, and/or so forth). In another example, a tablet computer or cellular telephone (cellphone) may provide the display component **60,** and processing may be performed by an application program ("app") running on the tablet or cellphone and/or with some processing performed by a hospital server in communication with the tablet computer or cell phone via WiFi, 4G, or another mobile communication network. It will be appreciated that these are not mutually exclusive options, e.g. in one cross-platform approach the patient status icons for all patients in the ICU is generated at a hospital server computer which is then transmitted to both the nurses' station and to each individual nurse's cell phone or other mobile device, where it is displayed. As yet another contemplated variant, if tablets, cellphones or other mobile devices are used as nurses' personal devices, and these devices have GPS, then the display can automatically update to show the patient status icon for the patient (or patients) in the hospital room currently being attended to by the nurse.

### EXAMPLES

A few clinical scenarios using the icons **10** are described. In a first example, the ICU director comes onto the unit. A digital screen representing the white board is displayed by the station. Reviewing the screen, the director observes the patient status icon **10** for each patient, and from those patient status icons readily apprehends that a few patients have some serious issues with multiple systems. The director also notes that he has six post-operative patients in the ICU, based upon the geometrical shapes of the patient status icons. The director talks to the nurse manager and follow-ups with the nurses assigned to the patients. The director also sees a few patients seem to be almost completely normal, thinking those patients might be candidates for transfer out of the ICU. This is discussed with the nurse manager and it is determined that two patients will be transferred out this morning.

In a second example, the ICU nurse is at the bedside of one of their patients. Looking at the clinical information system displayed on the tablet which is carried around, they notice that their other patient cardiovascular system just turned red. Immediately, the nurse clicks on the icons to drill down for the details. Nothing that the patient's blood pressure is lower than before, the nurse goes to the patient's bedside, assesses the patient further, and performs the appropriate action.

In a third example, a physician comes on duty in an electronic ICU (eICU) for the night shift and is receiving the status report from the physician going off-duty. They sit down at the ICU population screen on the electronic white board or nurses' station, which is covering 3 hospitals and 80 ICU patients. The physicians review the screen, and note the patient status icons **10.** Noting that 5 patients have multiple systems impacted, the physicians drill down for details to discuss the situation physician coming on duty. After the one physician leaves, the on duty physician calls the nurses at the hospital to discuss the patient, request further assessment, and writes orders as needed to address the patients' condition.

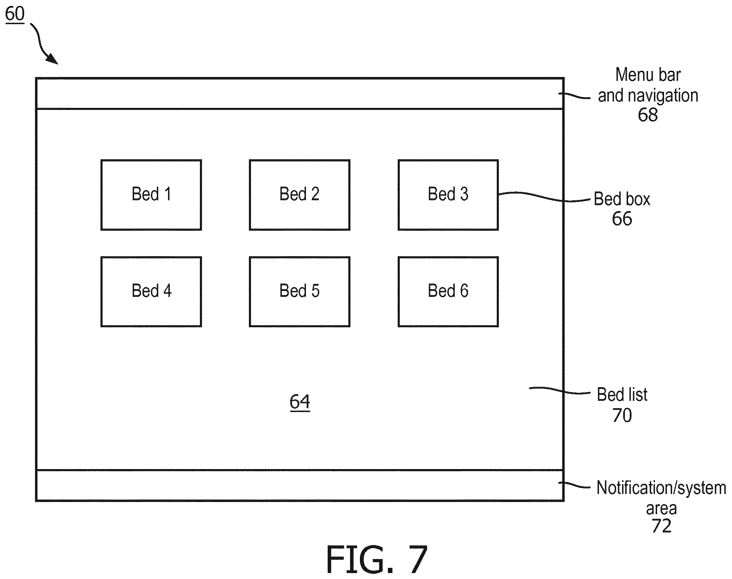

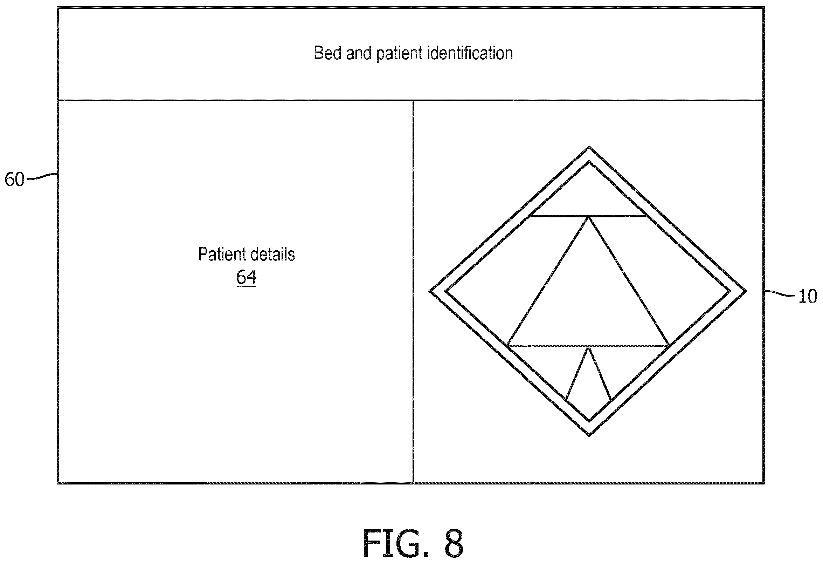

Once generated, the patient status icon **10** is displayed on the display device **60.** As shown in FIGURE 7, the white board or nurses' station displays other icons related to other patients on the display device **60** which in this example serves as the critical care unit population screen. The patient status icon is part of the clinical information system that includes a population or census screen **64,** which includes a set of bed boxes **66** on the census screen on the display device **60.** The bed box **66** contains patient information and status for the particular patient. Within the bed box is the patient status icon **10** for that patient. The patient status icon **10** updates real-time as new data comes concerning the patient's status. It can be used by the entire healthcare team. In a usual display on the display device **60,** the bed box **66** represents a bed in the care unit, most of these have an assigned patient. When a bed box **66** is selected on the display device **60,** the bed box opens up to include its own user interface components, one of which is the patient status icon **10,** as shown in FIGURE 8. Referring back to FIGURE 7, the display also includes a menu bar or navigation bar **68** that contains sub-components describing the name of the product, the user logged in, and other similar information. A bed list **70** contains all of the beds in the care unit as well as the unit name. A notification/system area window **72** includes sub components to display the date/time, notifications, and other information.

It will be appreciated that the illustrative computational, data processing or data interfacing components of the device **10** may be embodied as a non-transitory storage medium storing instructions executable by an electronic processor (e.g., the electronic processor **56**) to perform the disclosed operations. The non-transitory storage medium may, for example, comprise a hard disk drive, RAID, or other magnetic storage medium; a solid state drive, flash drive, electronically erasable read-only memory (EEROM) or other electronic memory; an optical disk or other optical storage; various combinations thereof; or so forth.

The population or census screen **64** includes a user interface (UI) that can be written (by way of non-limiting illustrative example) as an Angular JS/HTML5 application that can run on multiple types of devices such as clients, tablets, and others. The patient status icon **10** can be implemented as a component of that product. This framework can include a user interface that is displayed on the various devices. It is written in Angular JS, and includes the template and controller. Web Services which provide the data to the Client UI. Web Services access the database and update. This can be written with any standard programming language such as C#.

Portable components that contain data and are used to communicate data between the UI and the Web services. These may, for example, be represented as JSON objects. These portable components are bound to the UI components in the HTML5 Angular JS. Whenever data is changed in the portable component, the bound UI also changes. A portable component can be made of sub-portable components. For example, the patient portable component may have subcomponents for demographics, laboratory values, etc. In the illustrative UI example, there are various components which are each bound to their own portable component. Each UI component involves a Template which is an html like file and a Controller. The controller collects the POCOs (Plain Old CLR Objects) for the front end UI display and manages the interactions. One controller manages multiple Templates and POCOs such as all of the information displayed in the census screen **64.**

The patient status icon **10** is captured in an Angular JS file with a reference to a grouping of SVG (Scalable Vector Graphics) images. SVG are commonly used UI components and support software manipulation of the images. Every body system/concern in the Health Gem is an individual SVG image.

The patient status icon **10** is bound to a subcomponent portable component of a patient portable component. The patient portable component has subcomponents representing each body system and returns as a value the color of the component. The color is based upon clinical algorithms that generate a body system status based upon rules.

The controller who manages the templates obtains the portable component from the Web Services. In this case, the web services returns the patient portable component which has the information about the organ systems and their colors. The web services often use a data provider which accesses the information from the database using stored procedures against something like a SQL database.

The patient status icon **10** is designed to be used by multiple products. This includes the template and the portable component. These two pieces can be used in any product based upon web technologies.

For products which do not employ a web browser interface, the patient status icon **10** SVG can be encapsulated by software code, such as C# to make it a standard Microsoft UI component. As a UI component, it has basic functions to resize, reshape, etc. It also has specialized functions to set the color or status for individual body systems. In this version, a view is displayed to the user as a collection of Microsoft UI components. The view obtains it data and configuration information, called a model, from a backend service. The view could also use the same web services that are used in the web approach.

## Claims

1. A patient status monitoring apparatus (50) having at least one electronic processor **(56)** operatively connected with a display device **(60),** the patient status monitoring apparatus (50) being configured to perform a patient status monitoring method **(100)** comprising:
retrieving, from at least one database **(52),** medical information of a patient;
computing a state for each of a plurality of body systems or physiologies including at least a neurological body system, a cardiovascular body system, and a respiratory body system by applying clinical decision support processing **(54)** to the retrieved medical information;
displaying, on the display device (60), a patient status icon **(10)** with a plurality of user-selectable icon sections **(12, 14 16, 18, 20, 22, 24)** corresponding to the plurality of body systems or physiologies, each icon section being displayed with graphical coding comprising a color that represents the computed state of the corresponding body system or physiology of the patient;
receiving a user-selection of an icon section (12, 14, 16, 18, 20, 22, 24); and
displaying, on the display device (60), context-sensitive information of the selected body system or physiology,
wherein the icon sections corresponding to body systems form a map of the corresponding body systems in a coronal plane of the patient's body.

2. The patient status monitoring apparatus of claim 1, wherein the plurality of icon sections **(12, 14 16, 18, 20, 22, 24)** of the patient status icon **(10)** includes:
a cardiovascular icon section **(12)** corresponding to the cardiovascular body system which comprises an area centrally located on the icon;
a neurological icon section **(14)** corresponding to the neurological body system which comprises an area located above the cardiovascular icon section; and
a respiratory icon section **(16)** corresponding to the respiratory body system which includes left and right sub-sections comprising areas located left and right, respectively, of the cardiovascular icon section in the patient status icon.

3. The patient status monitoring apparatus of either one of claims 1 and 2, wherein:
the plurality of body systems or physiologies further includes a systemic infection physiology; and
the plurality of icon sections of the patient status icon **(10)** further includes a systemic infection icon section **(22)** corresponding to the systemic infection physiology wherein the systemic infection icon section comprises an outer border of the patient status icon **(10).**

4. The patient status monitoring apparatus of any one of claims 1-3, wherein:
the plurality of body systems or physiologies further inc000ludes at least one of (i) a gastrointestinal body system and (ii) a renal body system; and
the plurality of icon sections of the patient status icon further includes at least one of (i) a gastrointestinal icon section **(18)** corresponding to the gastrointestinal body system and located below the cardiovascular icon section and (ii) a renal icon section **(20)** corresponding to the renal body system and located below the cardiovascular icon section.

5. The patient status monitoring apparatus of any one of claims 1-4, wherein:
the plurality of body systems or physiologies further includes a hematology physiology; and
the plurality of icon sections of the patient status icon further includes a hematology icon section **(24)** corresponding to the hematology physiology wherein the hematology icon section comprises interior border lines **(26)** of the patient status icon **(10).**

6. The patient status monitoring apparatus of any one of claims 1-5, wherein the graphical coding complies with a coding scheme in which white represents a normal computed state of the corresponding body system or physiology, yellow represents a non-serious abnormal computed state of the corresponding body system or physiology, and red represents a serious abnormal computed state of the corresponding body system or physiology.

7. The patient status monitoring apparatus of claim 6, wherein the graphical coding complies with a coding scheme in which a background color other than white, yellow, or red represents an indeterminate computed state of the corresponding body system or physiology.

8. The patient status monitoring apparatus of any one of claims 1-7, wherein the patient status monitoring method **(100)** further comprises:
identifying, from the retrieved medical information, a medical therapy being delivered to the patient to treat a body system or physiology undergoing treatment of the plurality of body systems or physiologies;
wherein the displaying, on the display device **(60),** of the patient status icon **(10)** includes annotating the icon section **(12, 14 16, 18, 20, 22, 24)** corresponding to the body system or physiology undergoing treatment with a medical therapy indicator **(62)** representing the medical therapy.

9. The patient status monitoring apparatus of claim 8, wherein the medical therapy indicator **(62)** comprises one or more arrows representing a fluid flow through the patient produced by the medical therapy.

10. The patient status monitoring apparatus of any one of claims 1-9, wherein the displaying, on the display device **(60),** of the patient status icon **(10)** includes displaying the patient status icon having a shape indicative of a medical workflow state of the patient, the medical workflow state including at least one of a pre-operative workflow state, an operative state, a post-operative state, an adult patient state, a pediatric patient state, and a pregnancy state indicating a state of both a mother and a fetus.

11. A patient status monitoring method **(100),** comprising:
retrieving, from at least one database **(52),** medical information of a patient;
computing a state for each of a plurality of body systems or physiologies including at least a neurological body system, a cardiovascular body system, and a respiratory body system by applying clinical decision support (CDS) processing **(54)** to the retrieved medical information; and
displaying, on the display device **(60),** a patient status icon **(10)** with a plurality of user-selectable icon sections **(12, 14 16, 18, 20, 22, 24)** corresponding to the plurality of body systems or physiologies, each icon section being displayed with graphical coding comprising a color that represents encoding the computed state of the corresponding body system or physiology of the patient;
receiving a user-selection of an icon section (12, 14, 16, 18, 20, 22, 24); and
displaying, on the display device (60), context-sensitive information of the selected body system or physiology,
wherein the icon sections corresponding to body systems form a map of the corresponding body systems in a coronal plane of the patient's body.

12. The method **(100)** of claim 11, wherein the plurality of body systems or physiologies further includes at least a systemic infection physiology, a gastrointestinal body system, a renal body system, and a hematology physiology, and
wherein the displaying of the patient status icon **(10)** includes displaying the patient status icon with the plurality of icon sections **(12, 14 16, 18, 20, 22, 24)** including:
a cardiovascular icon section **(12)** corresponding to the cardiovascular body system which comprises a centrally located area of the icon;
a neurological icon section **(14)** corresponding to the neurological body system which comprises an area located above the cardiovascular icon section;
a respiratory icon section **(16)** corresponding to the respiratory body system which includes left and right sub-sections comprising areas located left and right, respectively, of the cardiovascular icon section in the patient status icon;
a systemic infection icon section **(22)** corresponding to the systemic infection physiology which comprises an outer border of the patient status icon;
a gastrointestinal icon section **(18)** corresponding to the gastrointestinal body system and comprising an area located below the cardiovascular icon section;
a renal icon section **(20)** corresponding to the renal body system and comprising an area located below the cardiovascular icon section; and
a hematology icon section **(24)** corresponding to the hematology physiology which comprises interior border lines **(26)** of the patient status icon.

13. The method **(100)** of either one of claims 11 and 12, wherein the displaying of the patient status icon **(10)** includes:
color coding each icon section **(12, 14 16, 18, 20, 22, 24)** with a color that represents the computed state of the corresponding body system or physiology of the patient, the color coding being in accord with a coding scheme in which:
white represents a normal computed state of the corresponding body system or physiology;
yellow represents a non-serious abnormal computed state of the corresponding body system or physiology;
red represents a serious abnormal computed state of the corresponding body system or physiology; and
a background color other than white, yellow, or red representing an indeterminate computed state of the corresponding body system or physiology.

14. The method **(100)** of any one of claims 11-13, further including:
identifying, from the retrieved medical information, a medical therapy being delivered to the patient to treat a body system or physiology undergoing treatment of the plurality of body systems or physiologies; and
annotating the icon section **(12, 14 16, 18, 20, 22, 24)** corresponding to the body system or physiology undergoing treatment with a medical therapy indicator **(62)** representing the medical therapy, the medical therapy icon comprises one or more arrows representing a fluid flow through the patient produced by the medical therapy.

15. The method **(100)** of any one of claims 11-14, wherein the displaying, on the display device **(60),** of the patient status icon **(10)** further includes:
displaying the patient status icon having a shape indicative of a medical workflow state of the patient, the medical workflow state including at least one of a pre-operative workflow state, an operative state, a post-operative state, an adult patient state, a pediatric patient state, and a pregnancy state indicating a state of both a mother and a fetus.

## Patentansprüche

1. Einrichtung (50) zur Patientenzustandsüberwachung, die mindestens einen elektronischen Prozessor **(56)** aufweist, der betriebsmäßig mit einer Anzeigevorrichtung **(60)** verbunden ist, wobei die Einrichtung (50) zur Patientenzustandsüberwachung so konfiguriert ist, dass sie ein Verfahren **(100)** zur Patientenzustandsüberwachung durchführt, das Folgendes umfasst:
Abrufen medizinischer Informationen eines Patienten aus mindestens einer Datenbank **(52);**
Berechnen eines Zustands für jedes einer Vielzahl von Körpersystemen oder Physiologien, die mindestens ein neurologisches Körpersystem, ein kardiovaskuläres Körpersystem und ein respiratorisches Körpersystem einschließen, durch Anwenden von Verarbeitung **(54)** zur Unterstützung klinischer Entscheidungen auf die abgerufenen medizinischen Informationen;
Anzeigen auf der Anzeigevorrichtung (60) eines Patientenzustandssymbols (10) mit einer Vielzahl von vom Benutzer auswählbaren Symbolabschnitten **(12, 14, 16, 18, 20, 22, 24),** die der Vielzahl von Körpersystemen oder Physiologien entsprechen, wobei jeder Symbolabschnitt mit grafischer Codierung angezeigt wird, die eine Farbe umfasst, die den berechneten Zustand des entsprechenden Körpersystems oder der Physiologie des Patienten darstellt;
Empfangen einer Benutzerauswahl eines Symbolabschnitts (12, 14, 16, 18, 20, 22, 24); und
Anzeigen auf der Anzeigevorrichtung (60) von kontextabhängigen Informationen zu dem ausgewählten Körpersystem oder der Physiologie,
wobei die den Körpersystemen entsprechenden Symbolabschnitte eine Karte der entsprechenden Körpersysteme in einer Frontalebene des Körpers des Patienten bilden.

2. Einrichtung zur Patientenzustandsüberwachung nach Anspruch 1, wobei die Vielzahl von Symbolabschnitten **(12, 14, 16, 18, 20, 22, 24)** des Patientenzustandssymbols **(10)** Folgendes einschließt:
einen kardiovaskulären Symbolabschnitt **(12),** der dem kardiovaskulären Körpersystem entspricht, der einen zentral auf dem Symbol gelegenen Bereich umfasst;
einen neurologischen Symbolabschnitt **(14),** der dem neurologischen Körpersystem entspricht, der einen Bereich umfasst, der oberhalb des kardiovaskulären Symbolabschnitts gelegen ist; und
einen Atmungssymbolabschnitt **(16),** der dem Atmungssystem des Körpers entspricht, der linke und rechte Unterabschnitte einschließt, die Bereiche umfassen, die jeweils links und rechts vom kardiovaskulären Symbolabschnitt im Patientenzustandssymbol gelegen sind.

3. Einrichtung zur Patientenzustandsüberwachung nach einem der Ansprüche 1 und 2, wobei:
die Vielzahl von Körpersystemen oder Physiologien weiter eine Physiologie systemischer Infektion einschließt; und
die Vielzahl von Symbolabschnitten des Patientenzustandssymbols **(10)** weiter einen Symbolabschnitt **(22)** für systemische Infektion einschließt, der der Physiologie systemischer Infektion entspricht, wobei der Symbolabschnitt für systemische Infektion einen äußeren Rand des Patientenzustandssymbols **(10)** umfasst.

4. Einrichtung zur Patientenzustandsüberwachung nach einem der Ansprüche 1-3, wobei:
die Vielzahl von Körpersystemen oder Physiologien weiter mindestens eines einschließt von (i) einem gastrointestinalen Körpersystem und (ii) einem renalen Körpersystem; und
die Vielzahl von Symbolabschnitten des Patientenzustandssymbols weiter mindestens eines einschließt von (i) einem gastrointestinalen Symbolabschnitt **(18),** der dem gastrointestinalen Körpersystem entspricht und unterhalb des kardiovaskulären Symbolabschnitts gelegen ist, und (ii) einem renalen Symbolabschnitt **(20),** der dem renalen Körpersystem entspricht und unterhalb des kardiovaskulären Symbolabschnitts gelegen ist.

5. Einrichtung zur Patientenzustandsüberwachung nach einem der Ansprüche 1-4, wobei:
die Vielzahl von Körpersystemen oder Physiologien weiter eine hämatologische Physiologie einschließt; und
die Vielzahl von Symbolabschnitten des Patientenzustandssymbols weiter einen Hämatologie-Symbolabschnitt **(24)** einschließt, der der hämatologischen Physiologie entspricht, wobei der Hämatologie-Symbolabschnitt innere Begrenzungslinien **(26)** des Patientenzustandssymbols **(10)** umfasst.

6. Einrichtung zur Patientenzustandsüberwachung nach einem der Ansprüche 1-5, wobei die grafische Kodierung ein Kodierungsschema erfüllt, bei dem Weiß einen normalen berechneten Zustand des entsprechenden Körpersystems oder der Physiologie darstellt, Gelb einen nicht schwerwiegenden abnormalen berechneten Zustand des entsprechenden Körpersystems oder der Physiologie darstellt und Rot einen schwerwiegenden abnormalen berechneten Zustand des entsprechenden Körpersystems oder der Physiologie darstellt.

7. Einrichtung zur Patientenzustandsüberwachung nach Anspruch 6, wobei die grafische Kodierung ein Kodierungsschema erfüllt, bei dem eine andere Hintergrundfarbe als Weiß, Gelb oder Rot einen unbestimmten berechneten Zustand des entsprechenden Körpersystems oder der Physiologie darstellt.

8. Einrichtung zur Patientenzustandsüberwachung nach einem der Ansprüche 1-7, wobei das Verfahren **(100)** zur Patientenüberwachung weiter Folgendes umfasst:
Identifizieren anhand der abgerufenen medizinischen Informationen einer medizinischen Therapie, die dem Patienten verabreicht wird, um ein Körpersystem oder eine Physiologie zu behandeln, die von der Vielzahl von Körpersystemen oder Physiologien einer Behandlung unterzogen wird;
wobei das Anzeigen des Patientenzustandssymbols **(10)** auf der Anzeigevorrichtung **(60)** Beschriften des Symbolabschnitts **(12, 14, 16, 18, 20, 22, 24),** der dem Körpersystem oder der Physiologie entspricht, das/die einer Behandlung unterzogen wird, mit einem medizinischen Therapieindikator **(62),** der die medizinische Therapie darstellt, einschließt.

9. Einrichtung zur Patientenzustandsüberwachung nach Anspruch 8, wobei der medizinische Therapieindikator **(62)** einen oder mehrere Pfeile umfasst, die einen durch die medizinische Therapie hervorgerufenen Fluidfluss durch den Patienten darstellen.

10. Einrichtung zur Patientenzustandsüberwachung nach einem der Ansprüche 1-9, wobei das Anzeigen des Patientenzustandssymbols **(10)** auf der Anzeigevorrichtung **(60)** Anzeigen des Patientenzustandssymbols umfasst, das eine Form aufweist, die einen medizinischen Arbeitsablaufzustand des Patienten angibt, wobei der medizinische Arbeitsablaufzustand mindestens eines einschließt von einem präoperativen Arbeitsablaufzustand, einem operativen Zustand, einem postoperativen Zustand, einem Zustand eines erwachsenen Patienten, einem Zustand eines pädiatrischen Patienten und einem Schwangerschaftszustand, der einen Zustand sowohl einer Mutter als auch eines Fötus angibt.

11. Verfahren **(100)** zur Patientenzustandsüberwachung, das Folgendes umfasst:
Abrufen medizinischer Informationen eines Patienten aus mindestens einer Datenbank **(52);**
Berechnen eines Zustands für jedes einer Vielzahl von Körpersystemen oder Physiologien, die mindestens ein neurologisches Körpersystem, ein kardiovaskuläres Körpersystem und ein respiratorisches Körpersystem einschließen, durch Anwenden von Verarbeitung **(54)** zur Unterstützung klinischer Entscheidungen (CDS) auf die abgerufenen medizinischen Informationen; und
Anzeigen auf der Anzeigevorrichtung **(60)** eines Patientenzustandssymbols **(10)** mit einer Vielzahl von vom Benutzer auswählbaren Symbolabschnitten **(12, 14, 16, 18, 20, 22, 24),** die der Vielzahl von Körpersystemen oder Physiologien entsprechen, wobei jeder Symbolabschnitt mit grafischer Codierung angezeigt wird, die eine Farbe umfasst, die Encodieren des berechneten Zustands des entsprechenden Körpersystems oder der Physiologie des Patienten darstellt;
Empfangen einer Benutzerauswahl eines Symbolabschnitts (12, 14, 16, 18, 20, 22, 24); und
Anzeigen auf der Anzeigevorrichtung (60) von kontextabhängigen Informationen zu dem ausgewählten Körpersystem oder der Physiologie,
wobei die den Körpersystemen entsprechenden Symbolabschnitte eine Karte der entsprechenden Körpersysteme in einer Frontalebene des Körpers des Patienten bilden.

12. Verfahren **(100)** nach Anspruch 11, wobei die Vielzahl von Körpersystemen oder Physiologien weiter mindestens eine Physiologie systemischer Infektion, ein gastrointestinales Körpersystem, ein renales Körpersystem und eine hämatologische Physiologie einschließt, und
wobei das Anzeigen des Patientenzustandssymbols **(10)** Anzeigen des Patientenzustandssymbols mit der Vielzahl von Symbolabschnitten **(12, 14, 16, 18, 20, 22, 24)** umfasst, die Folgendes einschließen:
einen kardiovaskulären Symbolabschnitt **(12),** der dem kardiovaskulären Körpersystem entspricht, der einen zentral gelegenen Bereich des Symbols umfasst;
einen neurologischen Symbolabschnitt **(14),** der dem neurologischen Körpersystem entspricht, der einen Bereich umfasst, der sich über dem kardiovaskulären Symbolabschnitt befindet;
einen Atmungssymbolabschnitt **(16),** der dem Atmungssystem des Körpers entspricht, der linke und rechte Unterabschnitte einschließt, die Bereiche umfassen, die jeweils links und rechts vom kardiovaskulären Symbolabschnitt im Patientenzustandssymbol gelegen sind;
einen Symbolabschnitt **(22)** für systemische Infektionen, der der Physiologie der systemischen Infektion entspricht, der einen äußeren Rand des Patientenzustandssymbols umfasst;
einen gastrointestinalen Symbolabschnitt **(18),** der dem gastrointestinalen Körpersystem entspricht und einen Bereich umfasst, der unterhalb des kardiovaskulären Symbolabschnitts gelegen ist;
einen renalen Symbolabschnitt **(20),** der dem renalen Körpersystem entspricht und einen Bereich umfasst, der unterhalb des kardiovaskulären Symbolabschnitts gelegen ist; und
einen Hämatologie-Symbolabschnitt **(24),** der der hämatologischen Physiologie entspricht, der innere Begrenzungslinien **(26)** des Patientenzustandssymbols umfasst.

13. Verfahren **(100)** nach einem der Ansprüche 11 und 12, wobei das Anzeigen des Patientenzustandssymbols **(10)** Folgendes einschließt:
Farbcodierung jedes Symbolabschnitts **(12, 14, 16, 18, 20, 22, 24)** mit einer Farbe, die den berechneten Zustand des entsprechenden Körpersystems oder der Physiologie des Patienten darstellt, wobei die Farbcodierung mit einem Codierungsschema in Einklang steht, in dem:
Weiß einen normalen berechneten Zustand des entsprechenden Körpersystems oder der Physiologie darstellt;
Gelb einen nicht schwerwiegenden abnormalen berechneten Zustand des entsprechenden Körpersystems oder der Physiologie darstellt;
Rot einen schwerwiegenden abnormalen berechneten Zustand des entsprechenden Körpersystems oder der Physiologie darstellt; und
eine andere Hintergrundfarbe als Weiß, Gelb oder Rot einen unbestimmten berechneten Zustand des entsprechenden Körpersystems oder der Physiologie darstellt.

14. Verfahren **(100)** nach einem der Ansprüche 11-13, das weiter Folgendes einschließt:
Identifizieren anhand der abgerufenen medizinischen Informationen einer medizinischen Therapie, die dem Patienten verabreicht wird, um ein Körpersystem oder eine Physiologie zu behandeln, die von der Vielzahl von Körpersystemen oder Physiologien einer Behandlung unterzogen wird; und
Beschriften des Symbolabschnitts **(12, 14, 16, 18, 20, 22, 24),** der dem Körpersystem oder der Physiologie entspricht, das/die einer Behandlung unterzogen wird, mit einem medizinischen Therapieindikator **(62),** der die medizinische Therapie darstellt, wobei das medizinische Therapiesymbol einen oder mehrere Pfeile umfasst, die einen Fluidfluss durch den Patienten darstellen, der durch die medizinische Therapie erzeugt wird.

15. Verfahren **(100)** nach einem der Ansprüche 11-14, wobei das Anzeigen des Patientenzustandssymbols **(10)** auf der Anzeigevorrichtung **(60)** weiter Folgendes einschließt:
Anzeigen des Patientenzustandssymbols, das eine Form aufweist, die einen medizinischen Arbeitsablaufzustand des Patienten angibt, wobei der medizinische Arbeitsablaufzustand mindestens eines einschließt von einem präoperativen Arbeitsablaufzustand, einem operativen Zustand, einem postoperativen Zustand, einem Zustand eines erwachsenen Patienten, einem Zustand eines pädiatrischen Patienten und einem Schwangerschaftszustand, der einen Zustand sowohl einer Mutter als auch eines Fötus angibt.

## Revendications

1. Appareil de surveillance d'état de patient (50) présentant au moins un processeur électronique (56) connecté fonctionnellement à un dispositif d'affichage (60), l'appareil de surveillance d'état de patient (50) étant configuré pour mettre en œuvre un procédé de surveillance d'état de patient (100) comprenant les étapes consistant à :
récupérer, à partir d'au moins une base de données (52), des informations médicales d'un patient ;
calculer un état pour chacun d'une pluralité de systèmes corporels ou de physiologies incluant au moins un système corporel neurologique, un système corporel cardiovasculaire et un système corporel respiratoire en appliquant un traitement d'aide à la décision clinique (54) aux informations médicales récupérées ;
afficher, sur le dispositif d'affichage (60), une icône d'état de patient (10) avec une pluralité de sections d'icône sélectionnables par un utilisateur (12, 14 16, 18, 20, 22, 24) correspondant à la pluralité de systèmes corporels ou de physiologies, chaque section d'icône étant affichée avec un codage graphique comprenant une couleur qui représente l'état calculé du système corporel ou de la physiologie correspondant(e) du patient ;
recevoir une sélection d'utilisateur d'une section d'icône (12, 14, 16, 18, 20, 22, 24) ; et
afficher, sur le dispositif d'affichage (60), des informations contextuelles du système corporel ou de la physiologie sélectionné(e),
dans lequel les sections d'icône correspondant aux systèmes corporels forment une carte des systèmes corporels correspondants dans un plan coronal du corps du patient.

2. Appareil de surveillance d'état de patient selon la revendication 1, dans lequel la pluralité de sections d'icône (12, 14, 16, 18, 20, 22, 24) de l'icône d'état de patient (10) inclut :
une section d'icône cardiovasculaire (12) correspondant au système corporel cardiovasculaire qui comprend une zone située au centre de l'icône ;
une section d'icône neurologique (14) correspondant au système corporel neurologique qui comprend une zone située au-dessus de la section d'icône cardiovasculaire ; et
une section d'icône respiratoire (16) correspondant au système corporel respiratoire qui inclut des sous-sections gauche et droite comprenant des zones situées respectivement à gauche et à droite de la section d'icône cardiovasculaire dans l'icône d'état de patient.

3. Appareil de surveillance d'état de patient selon l'une quelconque des revendications 1 et 2, dans lequel :
la pluralité de systèmes corporels ou de physiologies inclut en outre une physiologie d'infection systémique ; et
la pluralité de sections d'icône de l'icône d'état de patient (10) inclut en outre une section d'icône d'infection systémique (22) correspondant à la physiologie d'infection systémique, dans lequel la section d'icône d'infection systémique comprend une bordure extérieure de l'icône d'état de patient (10).

4. Appareil de surveillance d'état de patient selon l'une quelconque des revendications 1-3, dans lequel :
la pluralité de systèmes corporels ou de physiologies inclut en outre au moins un parmi : (i) un système corporel gastro-intestinal et (ii) un système corporel rénal ; et
la pluralité de sections d'icône de l'icône d'état de patient inclut en outre au moins une parmi (i) une section d'icône gastro-intestinale (18) correspondant au système corporel gastro-intestinal et située sous la section d'icône cardiovasculaire et (ii) une section d'icône rénale (20) correspondant au système corporel rénal et située sous la section d'icône cardiovasculaire.

5. Appareil de surveillance d'état de patient selon l'une quelconque des revendications 1-4, dans lequel :
la pluralité de systèmes corporels ou de physiologies inclut en outre une physiologie d'hématologie ; et
la pluralité de sections d'icône de l'icône d'état de patient inclut en outre une section d'icône d'hématologie (24) correspondant à la physiologie d'hématologie, dans lequel la section d'icône d'hématologie comprend des lignes de bordure intérieure (26) de l'icône d'état de patient (10).

6. Appareil de surveillance d'état de patient selon l'une quelconque des revendications 1-5, dans lequel le codage graphique est conforme à un schéma de codage dans lequel le blanc représente un état calculé normal du système corporel ou de la physiologie correspondant(e), le jaune représente un état calculé anormal non grave du système corporel ou de la physiologie correspondant(e), et le rouge représente un état calculé anormal grave du système corporel ou de la physiologie correspondant(e).

7. Appareil de surveillance d'état de patient selon la revendication 6, dans lequel le codage graphique est conforme à un schéma de codage dans lequel une couleur d'arrière-plan autre que le blanc, le jaune ou le rouge représente un état calculé indéterminé du système corporel ou de la physiologie correspondant(e).

8. Appareil de surveillance d'état de patient selon l'une quelconque des revendications 1-7, dans lequel le procédé de surveillance d'état de patient (100) comprend en outre l'étape consistant à :
identifier, à partir des informations médicales récupérées, une thérapie médicale administrée au patient pour traiter un système corporel ou une physiologie subissant un traitement parmi la pluralité de systèmes corporels ou de physiologies ;
dans lequel l'affichage, sur le dispositif d'affichage (60), de l'icône d'état de patient (10) inclut une annotation de la section d'icône (12, 14 16, 18, 20, 22, 24) correspondant au système corporel ou à la physiologie subissant un traitement avec un indicateur de thérapie médicale (62) représentant la thérapie médicale.

9. Appareil de surveillance d'état de patient selon la revendication 8, dans lequel l'indicateur de thérapie médicale (62) comprend une ou plusieurs flèches représentant un écoulement de fluide à travers le patient produit par la thérapie médicale.

10. Appareil de surveillance d'état de patient selon l'une quelconque des revendications 1-9, dans lequel l'affichage, sur le dispositif d'affichage (60), de l'icône d'état de patient (10) inclut un affichage de l'icône d'état de patient présentant une forme indiquant un état de flux de travail médical du patient, l'état de flux de travail médical incluant au moins un parmi un état de flux de travail préopératoire, un état opératoire, un état postopératoire, un état de patient adulte, un état de patient pédiatrique et un état de grossesse indiquant un état à la fois d'une mère et d'un fœtus.

11. Procédé de surveillance d'état de patient (100), comprenant les étapes consistant à :
récupérer, à partir d'au moins une base de données (52), des informations médicales d'un patient ;
calculer un état pour chacun d'une pluralité de systèmes corporels ou de physiologies incluant au moins un système corporel neurologique, un système corporel cardiovasculaire et un système corporel respiratoire en appliquant un traitement (54) d'aide à la décision clinique (CDS) aux informations médicales récupérées ; et
afficher, sur le dispositif d'affichage (60), une icône d'état de patient (10) avec une pluralité de sections d'icône sélectionnables par un utilisateur (12, 14 16, 18, 20, 22, 24) correspondant à la pluralité de systèmes corporels ou de physiologies, chaque section d'icône étant affichée avec un codage graphique comprenant une couleur qui représente un codage de l'état calculé du système corporel ou de la physiologie correspondant(e) du patient ;
recevoir une sélection d'utilisateur d'une section d'icône (12, 14, 16, 18, 20, 22, 24) ; et
afficher, sur le dispositif d'affichage (60), des informations contextuelles du système corporel ou de la physiologie sélectionné(e),
dans lequel les sections d'icône correspondant aux systèmes corporels forment une carte des systèmes corporels correspondants dans un plan coronal du corps du patient.

12. Procédé (100) selon la revendication 11, dans lequel la pluralité de systèmes corporels ou de physiologies inclut en outre au moins une physiologie d'infection systémique, un système corporel gastro-intestinal, un système corporel rénal et une physiologie d'hématologie, et
dans lequel l'affichage de l'icône d'état de patient (10) inclut un affichage de l'icône d'état de patient avec la pluralité de sections d'icône (12, 14 16, 18, 20, 22, 24) incluant :
une section d'icône cardiovasculaire (12) correspondant au système corporel cardiovasculaire qui comprend une zone de l'icône située au centre ;
une section d'icône neurologique (14) correspondant au système corporel neurologique qui comprend une zone située au-dessus de la section d'icône cardiovasculaire ;
une section d'icône respiratoire (16) correspondant au système corporel respiratoire qui inclut des sous-sections gauche et droite comprenant des zones situées respectivement à gauche et à droite de la section d'icône cardiovasculaire dans l'icône d'état de patient ;
une section d'icône d'infection systémique (22) correspondant à la physiologie d'infection systémique qui comprend une bordure extérieure de l'icône d'état de patient ;
une section d'icône gastro-intestinale (18) correspondant au système corporel gastro-intestinal et comprenant une zone située sous la section d'icône cardiovasculaire ;
une section d'icône rénale (20) correspondant au système corporel rénal et comprenant une zone située sous la section d'icône cardiovasculaire ; et
une section d'icône d'hématologie (24) correspondant à la physiologie d'hématologie qui comprend des lignes de bordure intérieure (26) de l'icône d'état de patient.

13. Procédé (100) selon l'une quelconque des revendications 11 et 12, dans lequel l'affichage de l'icône d'état de patient (10) inclut l'étape consistant à :
coder en couleur chaque section d'icône (12, 14 16, 18, 20, 22, 24) avec une couleur qui représente l'état calculé du système corporel ou de la physiologie correspondant(e) du patient, le codage couleur étant conforme à un schéma de codage dans lequel :
le blanc représente un état calculé normal du système corporel ou de la physiologie correspondant(e) ;
le jaune représente un état calculé anormal non grave du système corporel ou de la physiologie correspondant(e) ;
le rouge représente un état calculé anormal grave du système corporel ou de la physiologie correspondant(e) ; et
une couleur d'arrière-plan autre que le blanc, le jaune ou le rouge représentant un état calculé indéterminé du système corporel ou de la physiologie correspondant(e).

14. Procédé (100) selon l'une quelconque des revendications 11-13, incluant en outre les étapes consistant à :
identifier, à partir des informations médicales récupérées, une thérapie médicale administrée au patient pour traiter un système corporel ou une physiologie subissant un traitement parmi la pluralité de systèmes corporels ou de physiologies ; et
annoter la section d'icône (12, 14, 16, 18, 20, 22, 24) correspondant au système corporel ou à la physiologie subissant un traitement avec un indicateur de thérapie médicale (62) représentant la thérapie médicale, l'icône de thérapie médicale comprend une ou plusieurs flèches représentant un écoulement de fluide à travers le patient produit par la thérapie médicale.

15. Procédé (100) selon l'une quelconque des revendications 11-14, dans lequel l'affichage, sur le dispositif d'affichage (60), de l'icône d'état de patient (10) inclut en outre l'étape consistant à :
afficher l'icône d'état de patient présentant une forme indiquant un état de flux de travail médical du patient, l'état de flux de travail médical incluant au moins un parmi un état de flux de travail préopératoire, un état opératoire, un état postopératoire, un état de patient adulte, un état de patient pédiatrique et un état de grossesse indiquant un état à la fois d'une mère et d'un fœtus.
